# EUROPÄISCHE PATENTANMELDUNG

(11) **EP 3 733 142 A1**
(43) Veröffentlichungstag der Anmeldung: **04.11.2020**
(21) Anmeldenummer: 19172060.6
(22) Anmeldetag: 30.04.2019
(51) Int. Cl.: A61F 13/496, A61F 13/513, A61F 13/84

(54) **HYGIENEARTIKEL IN HÖSCHENFORM**

(71) Anmelder: W. Pelz GmbH & Co. KG, 23812 Wahlstedt/Holstein (DE)
(72) Erfinder:
(74) Vertreter: Hauck Patentanwaltspartnerschaft mbB

(57) **Zusammenfassung**

Hygieneartikel in Höschenform mit einem Vorderteil und einem Rückenteil sowie einen Vorderteil und Rückenteil miteinander verbindenden Schrittbereich, wobei Vorderteil und Rückenteil am oberen Rand eine Hüftöffnung umgrenzen und Vorderteil, Rückenteil und Schrittbereich an zwei seitlichen Rändern Beinöffnungen umgrenzen, im Schrittbereich ein Saugkern angeordnet ist und Vorderteil und Rückenteil parallel zum oberen Rand verlaufende und im entspannten Zustand den oberen Rand raffende Elastifizierungsmittel aufweisen,
dadurch gekennzeichnet, dass
im Bereich der Elastifizierungsmittel auf zumindest einer Seite des Rückenteiles und/oder des Vorderteiles ein Etikett angeordnet ist, das nur an seinen beiden seitlichen Rändern, die senkrecht zum oberen Rand verlaufen, mit dem Rückenteil und/oder dem Vorderteil verbunden ist, wobei das Etikett im gestrafften Zustand des Rückenteils und/oder des Vorderteiles flach an seiner Seite anliegt.

## Beschreibung

Die Erfindung betrifft einen Hygieneartikel in Höschenform.

Hygieneartikel in Höschenform (Pants) dienen dem Auffangen von Ausscheidungen des menschlichen Körpers wie Harn oder Stuhl. Sie werden insbesondere von Personen benutzt, die unter Inkontinenz leiden, vorwiegend von Senioren. Derartige Pants werden aus Fasermaterialien, insbesondere aus zellulosehaltigen und/oder Superabsorber enthaltenen Fasermaterialien, sowie aus Kunststofffolien hergestellt. Es handelt sich hierbei um bei Einmalartikel (*disposables*), die nach einmaligem Gebrauch weggeworfen werden. Die Hersteller sind bestrebt, die Eigenschaften von Pants denen von herkömmlicher Unterwäsche anzunähern, um Tragekomfort und Akzeptanz zu verbessern.

Grundsätzlich weisen Hygieneartikel in Höschenform einen Vorderteil, einen Rückenteil sowie einen Vorderteil und Rückenteil miteinander verbindenden Schrittbereich auf, wobei Vorderteil und Rückenteil am oberen Rand eine Hüftöffnung umgrenzen und Vorderteil, Rückenteil und Schrittbereich an zwei seitlichen Rändern Beinöffnungen umgrenzen, im Schrittbereich ein Saugkern angeordnet ist und Vorderteil und Rückenteil parallel zu den oberen Rändern verlaufende und im entspannten Zustand den oberen Rand raffende Elastifizierungsmittel aufweisen.

Die Elastifizierungsmittel sind elastische Fäden (z.B. Elastan-Fäden), die auf die Teile des Hygieneartikels im gespannten Zustand aufgebracht sind, sodass sie diesen im entspannten Zustand raffen. Parallel zum oberen Rand verlaufende Elastifizierungsmittel dienen dazu, den Hygieneartikel im Hüftbereich zu fixieren. Vorzugsweise sind zusätzlich an den Beinöffnungen Elastifizierungsmittel vorhanden, um den Hygieneartikel eng an die Beine des Trägers anzulegen.

Zur sicheren Fixierung des Hygieneartikels am Körper des Trägers sind die Elastifizierungsmittel deutlich stärker ausgeprägt als die Hosengummis bei herkömmlicher Unterwäsche, sodass die Hygieneartikel im entspannten Zustand insbesondere an der Hüftöffnung entsprechend stärker gerafft sind.

Hygieneartikel in Höschenform werden von den Herstellern in unterschiedlichen Größen, Schnitten und Passformen angeboten. Insbesondere gibt es spezielle Ausführungen für Frauen und für Männer. Deshalb besteht ein Bedürfnis, die Pants mit Kennzeichnungen zu versehen, welche die jeweilige Ausführungsart angeben.

Bekannt ist, die Kennzeichnungen auf die Pants aufzudrucken. Bevorzugt wird der Aufdruck nahe dem oberen Rand an der Innenseite des Rückenteiles aufgebracht, wie die Wäschelabel herkömmlicher Unterwäsche. Da der Aufdruck auf dem flachliegenden Rückenteil erfolgt, ist er bei gerafftem oberen Rand nicht oder nur schwer erkennbar. Folglich muss zur eindeutigen Identifizierung der Kennzeichnung der obere Rand straffgezogen werden. Zudem verstärkt die aufgedruckte Kennzeichnung die Anmutung eines Einmalartikels.

Davon ausgehend liegt der Erfindung die Aufgabe zugrunde, einen Hygieneartikel in Höschenform mit einer Kennzeichnung zur Verfügung zu stellen, die besser identifizierbar ist und weniger auf einen Einmalartikel hindeutet.

Die Aufgabe wird durch einen Hygieneartikel in Höschenform mit den Merkmalen von Anspruch 1 gelöst. Vorteilhafte Ausführungsarten des Hygieneartikels sind in Unteransprüchen angegeben.

Der erfindungsgemäße Hygieneartikel in Höschenform umfasst einen Vorderteil und einen Rückenteil sowie einen Vorderteil und Rückenteil miteinander verbindenden Schrittbereich, wobei Vorderteil und Rückenteil am oberen Rand eine Hüftöffnung umgrenzen und Vorderteil, Rückenteil und Schrittbereich an zwei seitlichen Rändem Beinöffnungen umgrenzen, im Schrittbereich ein Saugkern angeordnet ist und Vorderteil und Rückenteil parallel zum oberen Rand verlaufende und im entspannten Zustand den oberen Rand raffende Elastifizierungsmittel aufweisen,
dadurch gekennzeichnet, dass
im Bereich der Elastifizierungsmittel auf zumindest einer Seite des Rückenteiles und/oder des Vorderteiles ein Etikett angeordnet ist, das nur an seinen beiden seitlichen Rändern, die senkrecht zum oberen Rand verlaufen, mit dem Rückenteil und/oder dem Vorderteil verbunden ist, wobei das Etikett im gestrafften Zustand des Rückenteils und/oder des Vorderteils flach an seiner Seite anliegt.

Der erfindungsgemäße Hygieneartikel ist zur Kennzeichnung mit einem Etikett versehen. Das Etikett ist nur an den beiden seitlichen Rändern, die senkrecht zum oberen Rand verlaufen, mit dem Rückenteil und/oder dem Vorderteil verbunden, auf dem das Etikett angeordnet ist. Zudem ist das Etikett so mit dem Rückenteil und/oder Vorderteil verbunden, dass es im gestrafften Zustand des Rückenteils und/oder des Vorderteils flach an seiner Seite anliegt. Hierfür weisen die Stellen, an denen die beiden seitlichen Ränder des Etiketts mit dem Rückenteil und/oder Vorderteil verbunden sind, im gestrafften Zustand des Rückenteils und/oder Vorderteils einen Abstand voneinander auf, der dem Abstand der beiden seitlichen Ränder des flach ausgebreiteten Etiketts voneinander entspricht. Hierdurch wird zum einen erreicht, dass die Kennzeichnung auch dann lesbar ist, wenn der obere Rand des Hygieneartikels gerafft ist. Im gerafften Zustand des oberen Randes des Hygieneartikels wird nämlich das Etikett nicht gerafft, da es nur an seinen beiden seitlichen Rändern mit dem Rückenteil und/oder dem Vorderteil verbunden ist. Wenn das Etikett nur flexibel und nicht elastisch ist, kann es sich folglich nach innen auswölben, sodass es immer noch gut ablesbar ist. Falls das Etikett selber elastisch ist, kann es sich zusammenziehen, sodass der Aufdruck auf der flachen Seite weiterhin gut lesbar ist. Dies erleichtert die Handhabung des Hygieneartikels, da es für das Überprüfen der Kennzeichnung nicht erforderlich ist, diesen am oberen Rand straffzuziehen. Im gestrafften Zustand des oberen Randes des Hygieneartikels ist das Etikett ohnehin gut lesbar, da es dann flach an der Innenseite des Rückenteils und/oder des Vorderteiles anliegt. Zudem wird durch die Anordnung der Kennzeichnung an einem Etikett eine Annäherung an herkömmliche Unterwäsche erzielt, die vorzugsweise ebenfalls durch Etiketten gekennzeichnet wird. In Abweichung von herkömmlicher Unterwäsche ist das Etikett jedoch nicht flächig oder umlaufend um den äußeren Rand mit dem Höschen verbunden, sondern nur an den beiden seitlichen Rändern, um die Lesbarkeit im gerafften Zustand sicherzustellen.

Gemäß einer Ausführungsart der Erfindung ist das Etikett an seinen beiden seitlichen Rändern über die gesamte oder im Wesentlichen die gesamte Länge seiner beiden seitlichen Rändern mit dem Rückenteil und/oder dem Vorderteil verbunden. Gemäß einer weiteren Ausführungsart ist das Etikett an seinen beiden seitlichen Rändern ununterbrochen mit den Rückenteil und/oder dem Vorderteil verbunden. Gemäß einer weiteren Ausführungsart ist das Etikett an seinen beiden seitlichen Rändern jeweils entlang unterbrochener Linien und/oder punktuell mit dem Rückenteil und/oder dem Vorderteil verbunden.

Gemäß einer weiteren Ausführungsart ist das Etikett an der Innenseite des Rückenteils und/oder des Vorderteils angeordnet. Hierdurch wird eine weitere Annäherung an herkömmliche Unterwäsche erzielt. Zur weiteren Annäherung ist das Etikett auf einer Seite des Rückenteils angeordnet.

Mit der Anordnung des Etiketts im Bereich der Elastifizierungsmittel ist eine Anordnung in dem Bereich angesprochen, in dem der Hygieneartikel im entspannten Zustand durch die Elastifizierungsmittel gerafft wird. Somit ist hierdurch sowohl eine Anordnung des Etiketts auf derselben Höhe wie die Elastifizierungsmittel als auch eine Anordnung des Etiketts in einem Bereich unterhalb oder oberhalb der Elastifizierungsmittel einbezogen, der durch diese Elastifizierungsmittel im entspannten Zustand gerafft wird.

Gemäß einer weiteren Ausführungsart ist das Etikett unterhalb eines im oberen Randbereich des Rückenteiles und/oder des Vorderteils angeordneten elastischen Hüftbandes angeordnet, welches Elastifizierungsmittel bildet, die im entspannten Zustand den oberen Rand raffen. Die Anordnung unterhalb des elastischen Hüftbandes ist vorteilhaft für die Fixierung des Etiketts.

Gemäß einer weiteren Ausführungsart ist das Etikett aus einem textilen Material hergestellt. Hierdurch wird der Tragekomfort verbessert und eine weitere Annäherung an herkömmliche Unterwäsche erreicht. Hierfür ist gemäß einer weiteren Ausführungsart das Etikett aus einem Vlies hergestellt. Die Ausführung als Vlies ist auch vorteilhaft für das Aufdrucken einer Kennzeichnung.

Gemäß einer weiteren Ausführungsart ist das Etikett aus einem Material hergestellt, das keine oder im Wesentlichen keine Elastizität aufweist. Gemäß einer anderen Ausführungsart ist das Etikett aus einem elastischen Material hergestellt, das gedehnt auf das gestraffte Rückenteil und/oder Vorderteil aufgebracht ist, sodass es im gerafften Zustand des Rückenteils und/oder des Vorderteils flach an seiner Seite anliegt. Bei dieser Ausführungsart ist das Etikett unabhängig davon gut lesbar, ob der obere Rand gestrafft oder gerafft ist, da es stets flach an einer Seite des Rückenteils und/oder des Vorderteils anliegt. Hierdurch wird eine weitere Annäherung an herkömmliche Unterwäsche erreicht. Gemäß einer weiteren Ausführungsart weist das Etikett dieselbe oder im Wesentlichen dieselbe Elastizität wie der Hygieneartikel im Bereich der Elastifizierungsmittel auf, in dem das Etikett angeordnet ist.

Das Etikett wird vorzugsweise während der Produktion im gestrafften Zustand des Trägermaterials mit diesem verbunden. Dies kann erfolgen, bevor oder nachdem das Trägermaterial mit dem Elastifizierungsmittel versehen wird. Vorzugsweise wird das Elastifizierungsmittel bei seiner Anbringung am Trägermaterial elastisch gedehnt und das Trägermaterial gestrafft und wird in diesem Zustand das Etikett flach bzw. elastisch gedehnt auf das Trägermaterial aufgelegt und mit diesem an den seitlichen Rändern verbunden.

Gemäß einer weiteren Ausführungsart ist das Etikett bedruckt. Das Aufdrucken der Kennzeichnung ermöglicht eine besonders kostengünstige Herstellung. Grundsätzlich ist es aber auch möglich, das Etikett auf andere Weise mit einer Kennzeichnung zu versehen, beispielsweise durch Sticken. Ferner bezieht die Erfindung Ausführungen ein, bei denen das Etikett keine Kennzeichnung trägt, sodass es beispielsweise vor der Anwendung von Hand mit einer Kennzeichnung versehen werden kann.

Gemäß einer weiteren Ausführungsart ist das Etikett einfarbig oder mehrfarbig bedruckt.

Gemäß einer weiteren Ausführungsart ist das Etikett mittels mindestens eines Inkjet-Druckkopfes bedruckt. Dies ist vorteilhaft für eine schnelle und kostengünstige Herstellung.

Gemäß einer weiteren Ausführungsart ist das Etikett an den seitlichen Rändern mittels Ultraschall oder Hotmelt mit dem Rückenteil und/oder Vorderteil verbunden. Auch dies ist vorteilhaft für eine schnelle und/oder kostengünstige Herstellung.

Gemäß einer weiteren Ausführungsart umfasst die Kennzeichnung eine oder mehrere der folgenden Angaben: Größe, Saugstärke, Schnitt, Passform, Geschlecht des Trägers, Benutzungshinweis, Hersteller, Marke, Logo oder sonstige Grafik.

Die Erfindung wird nachfolgend anhand der anliegenden Bezeichnungen von Ausführungsbeispielen näher erläutert. In den Zeichnungen zeigen:
- Fig. 1: einen Hygieneartikel in Höschenform flach ausgebreitet in einer Draufsicht auf die Innenseite;
- Fig. 2: derselbe Hygieneartikel im gestrafften Zustand in einer Perspektivansicht schräg von oben;
- Fig. 3: derselbe Hygieneartikel in demselben Zustand in einem Schnitt entlang der Linie II-II von Fig. 1;
- Fig. 4: derselbe Hygieneartikel mit einem Etikett aus elastischem Material in gerafftem Zustand in demselben Schnitt wie in Fig. 3;
- Fig. 5: derselbe Hygieneartikel mit einem Etikett aus unelastischem und flexiblem Material im gerafften Zuschnitt in demselben Schnitt wie in Fig. 3;

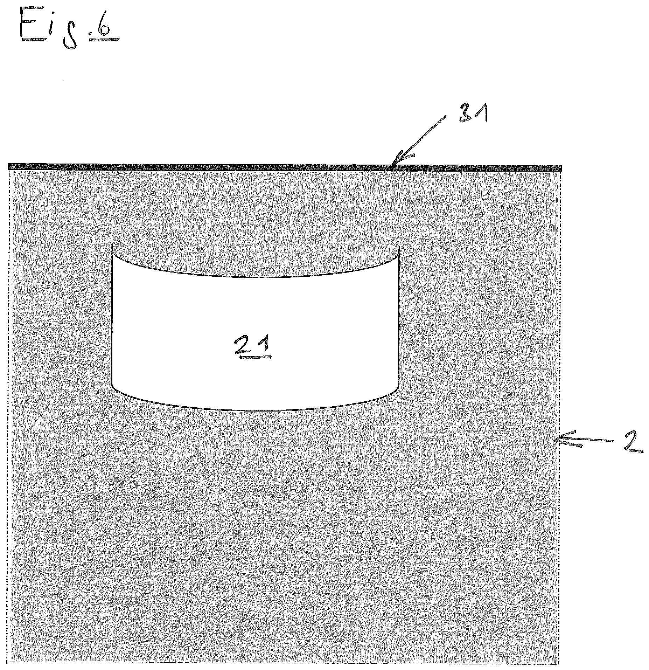

- Fig. 6: derselbe Hygieneartikel wie in Fig. 5 in gerafftem Zustand in einer vergrößerten Draufsicht auf das Etikett und die Innenseite des Rückenteils.

In der vorliegenden Anmeldung beziehen sich die Angaben "oben" und "unten" sowie "Vorderteil" und "Rückenteil" auf die Gebrauchsstellung des Hygieneartikels am Körper eines aufrecht stehenden Benutzers.

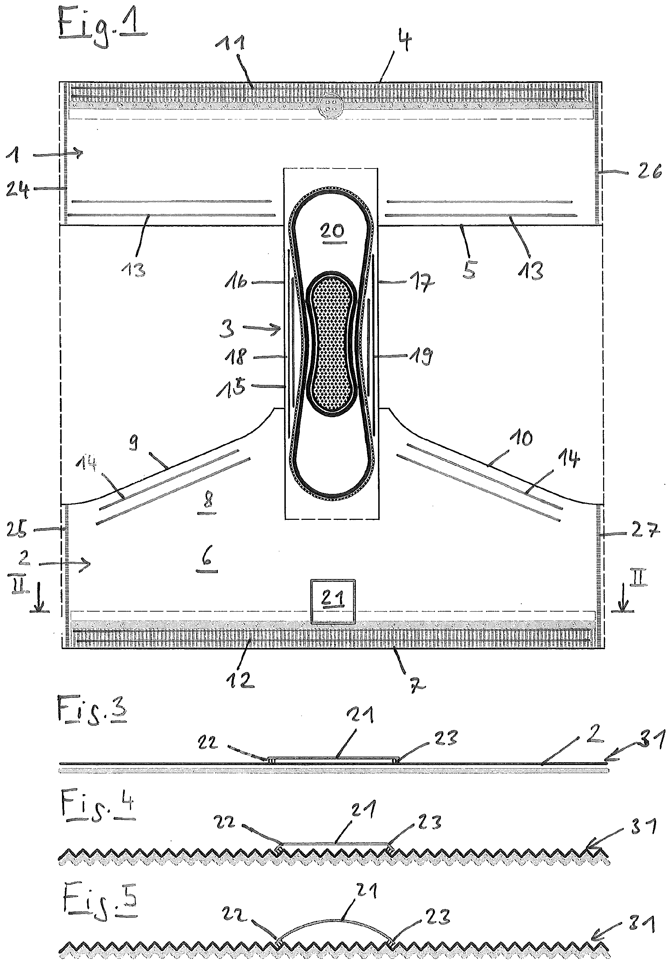

Gemäß Fig. 1 umfasst der Hygieneartikel einen Vorderteil 1, einen Rückenteil 2 sowie einen Vorderteil 2 und Rückenteil 3 miteinander verbindenden Schrittbereich 3.

Der Vorderteil 1 ist rechteckig mit einem oberen Rand 4 und einem dazu parallelen unteren Rand 5.

Der Rückenteil 2 hat einen rechteckigen oberen Abschnitt 6 mit einem oberen Rand 7 und einen im Wesentlichen trapezförmigen unteren Abschnitt 8 mit unteren Rändern 9, 10, die spitzwinklig zum oberen Rand 7 angeordnet sind und deren Abstand vom unteren Rand mit ihrer Annäherung an den Schrittbereich 3 zunimmt.

Der Vorderteil 1 und der Rückenteil 2 sind jeweils Laminate mit einer Wäscheschutzfolie an der Außenseite und einem Vlies an der Innenseite. Erste Elastifizierungsmittel 11, 12 sind neben den oberen Rändern 4, 7 und parallel zu diesen angeordnet. Zweite Elastifizierungsmittel 13, 14 sind neben den unteren Rändern 5, 9, 10 und parallel zu diesen angeordnet.

Der Schrittbereich 3 umfasst einen rechteckigen Trägerstreifen 15, der ebenfalls ein Laminat mit einer Wäscheschutzfolie an der Außenseite und einem Vlies an der Innenseite ist. Parallel zu den äußeren Rändern 16, 17 des Trägerstreifens 15 sind zwischen Wäscheschutzfolie und Vlies weitere zweite Elastifizierungsmittel 18, 19 angeordnet.

Die ersten Elastifizierungsmittel 11, 12 und die zweiten Elastifizierungsmittel 13, 14, 18, 19 sind jeweils durch Fäden aus einem elastomeren Material gebildet, beispielsweise durch Fäden aus Elastan (Lycra®). Die Fäden erstrecken sich parallel zu den jeweiligen Rändern, neben denen die betreffenden Elastifizierungsmittel angeordnet sind.

Auf der Innenseite des Trägerstreifens 15 ist ein Saugkern 20 zur Aufnahme von Trägerflüssigkeit angeordnet, der im Beispiel in der Draufsicht hantelförmig ausgebildet ist. Der Saugkern 20 umfasst saugfähiges zellulosehaltiges Material, das gegebenenfalls einen Superabsorber enthält. Das zellulosehaltige Material ist vorzugsweise auf der Innenseite durch eine Akquisitions- und Verteilschicht abgedeckt und in mindestens eine Vlieslage eingeschlagen, die vorzugsweise nur die seitlichen Randbereiche der Akquisitionsschicht überdeckt.

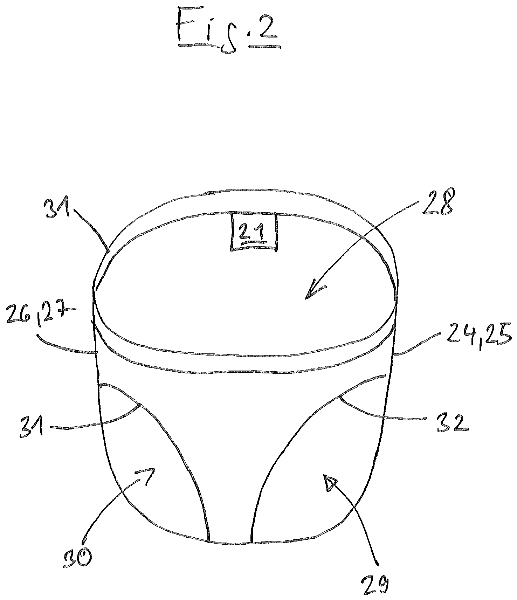

Auf der Innenseite des Rückenteiles 3 ist neben dem ersten Elastifizierungsmittel 12 ein Etikett 21 fixiert. Gemäß Fig. 2 ist das Etikett 21 ausschließlich an den beiden seitlichen Rändern 22, 23 an dem Rückenteil 2 fixiert, die senkrecht zum oberen Rand 7 des Rückenteiles 3 erstreckt sind. Die Fixierung erfolgt beispielsweise mittels Hotmelt oder Ultraschall.

Das Etikett 21 ist auf der Innenseite mit einem Aufdruck versehen, der beispielsweise Angaben über die Größe, die Stärke und den Schnitt sowie das Geschlecht des vorgesehenen Benutzers umfassen.

In Fig. 1 ist ein Zwischenprodukt des Hygieneartikels gezeigt. Zur Fertigstellung des Hygieneartikels müssen die benachbarten äußeren Ränder 24 und 25 sowie 26 und 27 des Vorderteils 1 und des Rückenteils 2 noch miteinander verbunden werden. Die Verbindung erfolgt beispielsweise durch Übereinanderlegen und Verschweißen mittels Ultraschall der benachbarten äußeren Ränder 24 und 25 sowie 26 und 27 von Vorderteil 1 und Rückenteil 2 miteinander.

Gemäß Fig. 2 umgrenzen im fertigen Hygieneartikel die oberen Ränder 4, 7 von Vorderteil 1 und Rückenteil 2 eine Hüftöffnung 28 und die unteren Ränder 5, 9, 10 von Vorderteil 1 und Rückenteil 2 und der diese jeweils verbindende äußere Rand 16, 17 des Trägerstreifens 15 Beinöffnungen 29, 30. Insgesamt bilden somit die oberen Ränder 4, 7 von Vorderteil 1 und Rückenteil 2 den die Hüftöffnung 28 umgrenzenden oberen Rand 31 des Hygieneartikels und die unteren Ränder 5, 9, 10 von Vorderteil 1 und Rückenteil 2 sowie die diese verbindenden äußeren Ränder 16, 17 des Trägerstreifens die Beinöffnungen 29, 30 umgrenzende seitliche Ränder 32, 33 des Hygieneartikels.

Im gestrafften Zustand des Rückenteils 2 ist auch das Etikett 21 gestrafft und liegt flach an der Innenseite des Rückenteiles 2 an. Infolgedessen sind die Angaben auf dem Etikett 21 gut lesbar. Dies verdeutlicht Fig. 3.

Im entspannten Zustand des Hygieneartikels ziehen sich insbesondere die ersten Elastifizierungsmittel 11, 12 zusammen, sodass der Hygieneartikel insbesondere am oberen Rand 31 gerafft wird.

Bei dem Ausführungsbeispiel von Fig. 4 besteht das Etikett 21 aus einem elastischen Material, das elastisch gedehnt auf den Hygieneartikel in dem gestrafften Zustand gemäß Fig. 1 aufgebracht worden ist. Infolgedessen bleibt bei gerafftem oberen Rand 31 des Hygieneartikels das Etikett 21 straff und der Aufdruck ist weiterhin gut lesbar.

Bei dem Ausführungsbeispiel von Fig. 5 besteht das Etikett 21 aus einem flexiblen, aber unelastischen oder im Wesentlichen unelastischen Material. Das Etikett 21 ist im gestrafften Zustand des Hygieneartikels gemäß Fig. 1 flach liegend auf seine Innenseite aufgebracht und daran befestigt. Infolgedessen wölbt es sich bei gerafftem Hygieneartikel leicht zur Innenseite hin aus, wie in Fig. 4 und 5 gezeigt. Dennoch ist der Aufdruck auf dem Etikett gut lesbar.

### Bezugszeichenliste:

- 1: Vorderteil
- 2: Rückenteil
- 3: Schrittbereich
- 4: oberer Rand
- 5: unterer Rand
- 6: oberer Abschnitt
- 7: oberer Rand
- 8: unterer Abschnitt
- 9, 10: unterer Rand
- 11, 12: erste Elastifizierungsmittel
- 13, 14: zweite Elastifizierungsmittel
- 15: Trägerstreifen
- 16, 17: äußerer Rand
- 18, 19: zweite Elastifizierungsmittel
- 20: Saugkern
- 21: Etikett
- 22, 23: seitlicher Rand
- 24 bis 27: äußerer Rand
- 28: Hüftöffnung
- 29, 30: Beinöffnung
- 31: oberer Rand
- 32, 33: seitlicher Rand

## Patentansprüche

1. Hygieneartikel in Höschenform mit einem Vorderteil (1) und einem Rückenteil (2) sowie einen Vorderteil (1) und Rückenteil (2) miteinander verbindenden Schrittbereich (3), wobei Vorderteil (1) und Rückenteil (2) am oberen Rand (30) eine Hüftöffnung (28) umgrenzen und Vorderteil (1), Rückenteil (2) und Schrittbereich (3) an zwei seitlichen Rändern (32, 33) Beinöffnungen (29, 30) umgrenzen, im Schrittbereich (3) ein Saugkern (20) angeordnet ist und Vorderteil (1) und Rückenteil (2) parallel zum oberen Rand (30) verlaufende und im entspannten Zustand den oberen Rand raffende Elastifizierungsmittel (11, 12) aufweisen,
**dadurch gekennzeichnet, dass**
im Bereich der Elastifizierungsmittel (11, 12) auf zumindest einer Seite des Rückenteiles (2) und/oder des Vorderteiles (1) ein Etikett (21) angeordnet ist, das nur an seinen beiden seitlichen Rändern (22, 23), die senkrecht zum oberen Rand (30) verlaufen, mit dem Rückenteil (2) und/oder dem Vorderteil (1) verbunden ist, wobei das Etikett (21) im gestrafften Zustand des Rückenteils (2) und/oder des Vorderteiles (1) flach an seiner Seite anliegt.

2. Hygieneartikel nach Anspruch 1, bei dem das Etikett (21) auf der Innenseite des Rückenteils (2) und/oder des Vorderteils (1) angeordnet ist.

3. Hygieneartikel nach Anspruch 1 oder 2, bei dem das Etikett (21) auf einer Seite des Rückenteiles (2) angeordnet ist.

4. Hygieneartikel nach einem der Ansprüche 1 bis 3, bei dem das Etikett (21) unterhalb eines im oberen Randbereich des Rückenteils (2) und/oder des Vorderteiles (1) angeordneten elastischen Hüftbandes angeordnet ist.

5. Hygieneartikel nach einem der Ansprüche 1 bis 4, bei dem das Etikett (21) aus einem textilen Material hergestellt ist.

6. Hygieneartikel nach Anspruch 5, bei dem das Etikett (21) aus einem Vlies hergestellt ist.

7. Hygieneartikel nach einem der Ansprüche 1 bis 6, bei dem das Etikett (21) aus einem elastischen Material hergestellt ist, das gedehnt auf das gestraffte Rückenteil (2) und/oder Vorderteil (1) aufgebracht ist, sodass es im gerafften Zustand des Rückenteils (2) und/oder Vorderteils (1) flach an seiner Seite anliegt.

8. Hygieneartikel nach einem der Ansprüche 1 bis 7, bei dem das Etikett (21) bedruckt ist.

9. Hygieneartikel nach Anspruch 8, bei dem das Etikett (21) einfarbig oder mehrfarbig bedruckt ist.

10. Hygieneartikel nach Anspruch 8 oder 9, bei dem das Etikett (21) mittels mindestens eines Inkjet-Druckkopfes bedruckt ist.

11. Hygieneartikel nach einem der Ansprüche 1 bis 10, bei dem das Etikett(21) an den seitlichen Rändern (22, 23) mittels Ultraschall oder Hotmelt mit dem Rückenteil und/oder Vorderteil verbunden ist.

12. Hygieneartikel nach einem der Ansprüche 1 bis 11, bei dem das Etikett (21) eine oder mehrere der folgenden Kennzeichnungen umfasst: Größe, Saugstärke, Schnitt, Passform, Geschlecht des Trägers, Gebrauchshinweis, Entsorgungshinweis, Hersteller, Marke, Logo oder sonstige Grafik.
